(19)

Europäisches
Patentamt
European
Patent Office
Office européen
des brevets

(11) **EP 3 388 837 A1**

(12) **EUROPEAN PATENT APPLICATION**

(43) Date of publication:
17.10.2018 Bulletin 2018/42

(51) Int Cl.:
*G01N 33/576* (2006.01)

(21) Application number: 17166600.1

(22) Date of filing: 13.04.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Universität Regensburg - Universitätsklinikum Regensburg 93053 Regensburg (DE)**

(72) Inventors:
• **Hutchinson, James Alexander**
**93051 Regensburg (DE)**
• **Geissler, Edward**
**93173 Wenzenbach (DE)**
• **Werner, Jens**
**93049 Regensburg (DE)**

(74) Representative: **Engelhard, Markus**
**Boehmert & Boehmert**
**Anwaltspartnerschaft mbB**
**Pettenkoferstrasse 22**
**80336 München (DE)**

(54) **A METHOD FOR PREDICTING TIME-TO-VIRAL CLEARANCE IN PATIENTS WITH CHRONIC HEPATITIS C VIRUS (HCV) INFECTION UNDER DIRECT ACTING ANTIVIRAL (DAA) THERAPY**

(57) The present invention relates to measurement of immunological parameters as a means to predict time-to-viral clearance in patients with chronic Hepatitis C Virus (HCV) infection under direct acting antiviral (DAA) therapy. The present invention provides a principle and methods for stratifying individuals into suitable treatment groups and personalized methods of treatment for such individuals.

EP 3 388 837 A1

**Description**

Field of the invention

The present invention relates to the treatment of chronic hepatitis C virus (HCV) infection with direct acting antiviral (DAA) therapeutics. In particular, the present invention relates to an optimized treatment of chronic hepatitis C virus (HCV) infections with DAA therapeutics based on immunological parameters of individual patients, wherein the response rate of patients to DAA and/or the time-to-viral clearance can be predicted.

Background

Recent introduction of all-oral direct acting antiviral (DAA) treatment has revolutionized care of patients with chronic hepatitis C virus (HCV) infection. Regrettably, the high cost of DAA treatment is burdensome for healthcare systems and may be prohibitive for some patients who would otherwise benefit. Currently, the DAA treatment is administered for at least 12 weeks. The daily costs for such treatment, e.g., with DAA Sofosbuvir, amount to about USD 1,000. Treatment with both, sofosbuvir and ledipasvir amount to daily costs of USD 1,125. It is estimated that the overall costs for the treatment of the approximately one million patients alone in the USA that receive DAA therapy will be an enormous burden for the overall health budget. Understanding how patient-related factors influence individual responses to DAA treatment may lead to more efficient prescription of the drugs for a necessary period of time, thereby hopefully decreasing the overall costs.

Direct-acting antiviral agents fall into four classes defined by the steps in the life cycle of HCV that they disrupt - namely, NS3/4A protease inhibitors (e.g. paritaprevir), NS5B nucleoside polymerase inhibitors (e.g. sofosbuvir), NS5B non-nucleoside polymerase inhibitors (e.g. dasabuvir), and NS5A inhibitors (e.g. ledipasvir, daclatasvir). These drugs are administered in combinations chosen on the basis of viral genotype (GT) and residual liver function. Following AASLD/IDSA guidelines,(6) patients with GT1 (4/5/6) infection without cirrhosis should be treated for 12 weeks with either a combination of ledipasvir (LDV) and sofosbuvir (SOF), or a combination of ritonavir-boosted paritaprevir, ombitasvir and dasabuvir, optionally with ribavirin (RBV). Alternatively, daclatasvir (DCV) may be used in combination with SOF for 12 weeks. Patients with GT2 infection should be treated with SOF and RBV for 12 weeks or, like patients with GT3 infection, they could be treated with a combination of DCV and SOF for 12 weeks.(7) Currently, the only exception to 12-week courses of treatment is a recommendation made in the EASL guidelines that DAA therapy may be shortened to 8 weeks in previously untreated patients with GT1 infection, who are without cirrhosis and whose baseline HCV RNA level is below 6 million IU/ml.(8) From a health-economics standpoint, it would be valuable if other subgroups of patients who only require short-course DAA therapy could be easily and reliably identified.

Patients with chronically HCV-infected hepatocytes frequently possess virus-specific T cells that are present in the affected organ, the liver and related structures. However, these T cells are very rare and obtaining these cells for further analysis is extremely difficult. For example, measuring them in order to predict a patient's response to a given anti-viral treatment may require their direct sampling from the affected organ. This would be an unjustifiable intervention for the patient. Therefore, alternative ways of correlating the likelihood of a patient's clinical response under a respective anti-viral treatment to the patient's systemic immunological condition would be preferable, which do not require the above invention.

According to the present invention, patients receiving DAA treatment were comprehensively monitored to identify immunologically-related biomarkers from peripheral blood that accurately predicted early HCV RNA negativity. This led to a predictive model based on CD3+ T cell and naive CD8+ T cell frequencies that accurately classified patients as "fast" responders (i.e. HCV RNA negative within 4 weeks of commencing DAA therapy) in a surprisingly high number of of cases.

Description of the figures

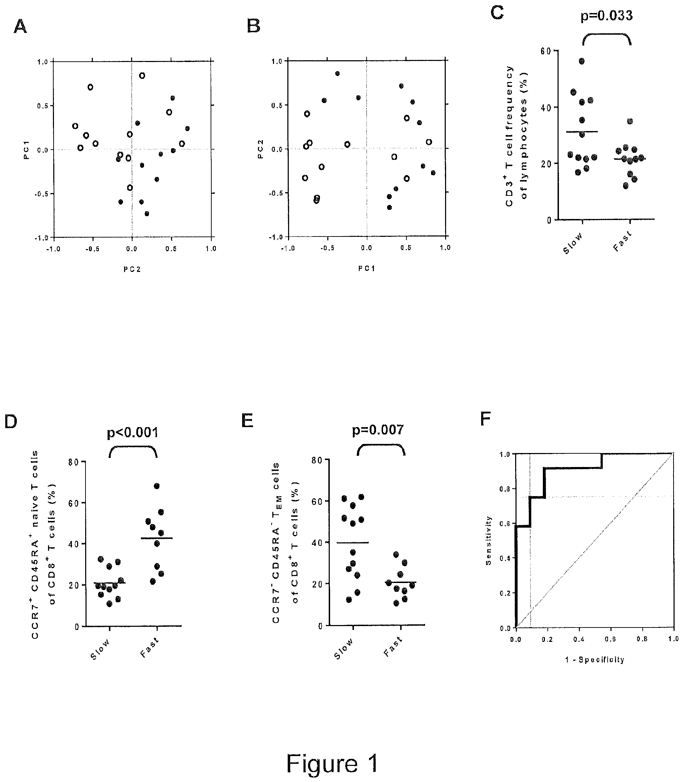

Figure 1 - Statistical analyses of flow cytometry dataset: (A) The first two principal components (PC1, PC2) of entire dataset of baseline standardized leucocyte frequencies partly distinguished fast (open circles) and slow (solid circles) responders. (B) After filtering the baseline dataset for leucocyte populations whose frequencies were significantly influenced by DAA treatments, the first two principal components of standardized leucocyte frequencies separated fast (open circles) and slow (solid circles) responders. (C) Comparison of baseline CD3+ T cell frequency between fast and slow responders. (D) Comparison of baseline naïve CD8+ T cell frequency between fast and slow responders. (E) Comparison of baseline CD8+ TEM cell frequency between fast and slow responders. (F) A cut-off value of 0.66 for scoring fast and slow responders was determined using receiver operator characteristic (ROC) curve analysis, which gave a test sensitivity of 75.0% and specificity of 91.0%.

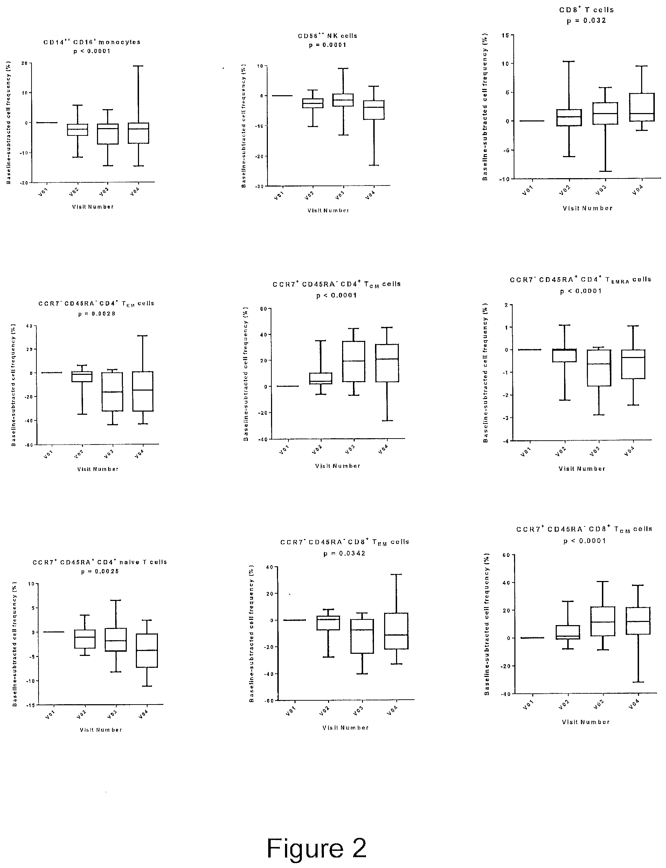

Figure 2 - Graphical summary of significant changes in leucocyte subset frequency between baseline and subsequent visits. Biologically relevant changes in leucocyte subset frequencies in individual patients may be relatively small compared to biological variation between individuals, comparing individual baseline-subtracted values between serial samples can be a sensitive approach for detecting immunological changes. Significant changes in baseline-subtracted leucocyte subset frequencies between visits were identified by pairwise significance testing.

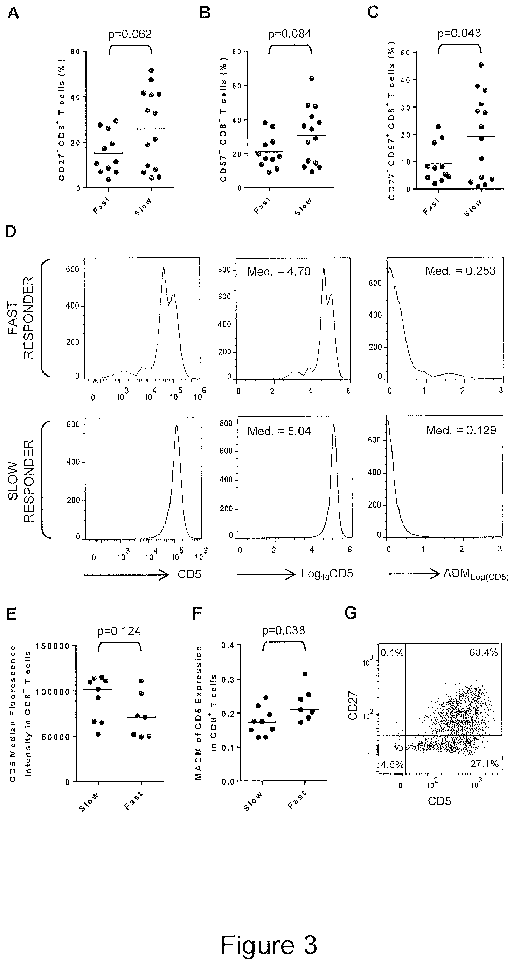

Figure 3 - Generalized activation of peripheral blood $CD8^+$ T cells in slow responders. (A) Frequencies of $CD27^-$ $CD8^+$ T cells in fast- and slow- responders. (B) Frequencies of $CD57^+$ $CD8^+$ T cells in fast- and slow- responders. (C) Frequencies of chronically activated $CD27^-$ $CD57^+$ $CD8^+$ T cells in fast- and slow- responders. (D) Visual representation of the method used to estimate the dispersion of CD5 expression in $CD8^+$ T cells. The examples represent one fast and one slow responder from samples of n=7 and n=9, respectively. The first pair of histograms shows CD5 fluorescence intensities of>1 plotted on a log-axis. The second pair of histograms shows $\log_{10}$-transformed values plotted on a linear-axis and their respective medians (Med.). The third pair of histograms shows absolute deviation of $\log_{10}$-transformed values from median (ADM) and the respective medians (MADM). (E) CD5 expression by $CD8^+$ T cells from fast- and slow- responders estimated by median fluorescence intensity. (F) $MADM_{Log(CD5)}$ in $CD8^+$ T cells from fast- and slow- responders. Assessing the spread of CD5 expression in $CD8^+$ T cells discriminates better between fast- and slow- responders. (G) Down-regulation of CD5 expression appears to be confined to $CD27^-$ $CD8^+$ T cells.

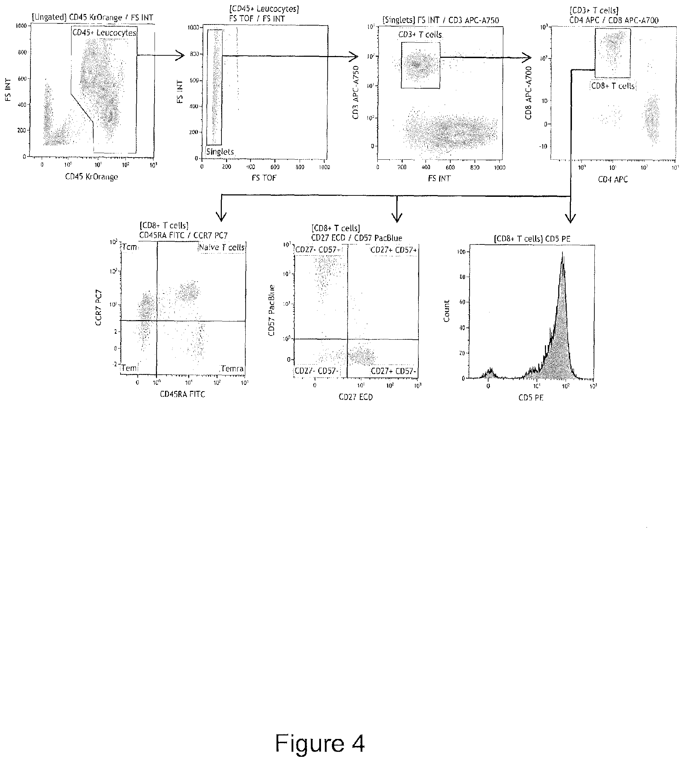

Figure 4 - Consolidated flow cytometry panel and recommended gating strategy. Example data from one patient with chronic HCV infection is shown.

## Embodiments and definitions

Throughout the following description, embodiments are disclosed that occasionally refer to lists or groups of numbers or other members. Occasionally, embodiments refer to one or even more other embodiments that may also comprise one or more groups or lists comprising individual members. It is explicitly contemplated that any combination of members of lists referred to below in different embodiments constitutes a further individual embodiment with the proviso that such combination makes technical sense to a person skilled in the art.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

According to the present invention, the expression "DAA therapy" or "DAA-treatment" relates to a therapy of an infection with HCV using any of the following direct acting antiviral compounds either alone or in combination selected from the following group: Daclatasvir, sofosbuvir, ledipasvir, ritonavir, paritaprevir, ombitasvir, dasabuvir, etc.

According to the present invention, the term "individual" relates to chronically HCV-infected subjects, in particular human patients.

According to the present invention, the term "HCV" relates to any genotype of said virus, e.g. genotypes 1a, 1b, 2, 3, 4, 5 and 6.

According to the present invention, the term "baseline frequency" indicates the frequency of a parameter of interest before the treatment of an individual with a given DAA or a combination thereof. A parameter of interest may, for example, be the viral load or the percentage frequency of a given cell type as described by respective phenotypic characteristics.

According to the present invention, the term "fast response rate" or "fast responder" describes a chronically HCV-infected patient that has a statistically significant probability of experiencing HCV negativity (i.e. no HCV RNA detection with currently available detection methods, e.g. as measured by PCR, particularly by quantitative real-time PCR in peripheral blood samples, e.g. in samples of at least 5 ml EDTA blood or whole blood) after four weeks of treatment with DAA therapy. The classification of a patient as "fast responder" is determined by measurement of the herein disclosed markers, i.e. the frequencies and/or absolute numbers of certain cell populations detected in a blood sample, e.g. a peripheral blood sample. Subsequently, based on these measurements, a logistic regression analysis is performed and the Odds ratios are calculated. A cut-off threshold may be assigned to the according to the test sensitivity and/or test specificity of the respective measurement method. Further, the probability that a patient may be classified into the group of "fast responders" in respect of the "time-to-viral clearance" ("fast responder status") is calculated using parameters obtained by logistic regression analysis.

The expression "time-to-viral clearance" indicates the period until no HCV RNA detection with currently available detection methods, e.g. as measured by PCR, particularly by quantitative real-time PCR in peripheral blood samples, e.g. in samples of at least 5 ml EDTA blood or whole blood is possible. Using a standard PCR-based test, fewer than 12 copies per milliliter blood is considered to be clinically clear of HCV.

According to the present invention, the term "slow response rate" or "slow responder" describes a chronically HCV-infected patient that has a statistically significant probability of experiencing continuing HCV infection (i.e. HCV RNA detected with currently available detection methods, e.g. as measured by PCR, particularly by quantitative real-time PCR in peripheral blood samples, e.g. in samples of at least 5 ml EDTA blood or whole blood) after four weeks of treatment with DAA therapy. The classification of a patient as "slow responder" is determined by measurement of the herein disclosed markers, i.e. the frequencies and/or absolute numbers of certain cell populations detected in a blood sample, e.g. a peripheral blood sample. Subsequently, based on the measurements, a logistic regression analysis is performed and the Odds ratios are calculated. A cut-off threshold may be assigned to the according to the test sensitivity and/or test specificity of the respective measurement method. Further, the probability that a patient may be classified into the group of "slow responders" in respect of the "time-to-viral clearance" ("fast responder status") is calculated using logistic regression analysis.

According to the present invention, a binary logistic regression analysis (or other statistical method of correlation) of baseline leucocyte frequencies can be made to calculate Odds ratios and 95% confidence intervals, respectively, in order to compare the respective groups of individuals and determine the strength of association of the presence or absence of an outcome with a population of individuals. This binary logistic model is used to estimate the probability of a binary response based on one or more predictor (or independent) variables (features) and to say that the presence of a risk factor increases the probability of a given outcome by a specific percentage as known in the art (*cf.* Whitlock and Schluter, 2nd Ed., The analysis of biological data, June 2014).

According to the invention, the "statistical significant probability" refers to an above-threshold value for the calculated probability of a chronically HCV-infected patient being found to be a "fast responder" under treatment. The probability of an individual chronically HCV-infected patient being found to be a "fast responder" under treatment can be calculated from that patient's frequency and/or absolute numbers of CD3$^+$ and CD8$^+$ naïve T cells using coefficients taken from a binary logistical regression model. As used herein, a threshold (or "cut-off" value) for evaluating whether a patient's probability of being a "fast responder" classifies them as either a "fast" or "slow" responder is derived from receiver-operator curve (ROC) analysis. In embodiments of the present invention, said cut-off value is 0.66 given the chosen level of test specificity, but other cut-off thresholds may be asserted as understood by a skilled person, e.g. a biostatistician or clinical statistician. The test sensitivity obtained in the ROC curve analysis is a mathematical function of test specificity. In embodiments of the present invention, given that a high specificity of 91.0% was set, test sensitivity was 75.0%.

The test is potentially useful if it predicts responder status more often than the "null assumption" (or "no information model") that asserts either none or all individuals are fast responders, whichever is the more prevalent condition. The prevalence of fast responders depends upon the particular subset of patients under investigation - specifically, whether they are "hard-to-treat" cases or not. In our general cohort, under the null assumption, 60% of patients would have been correctly classified, whereas the test allowed an 86.6% overall correct classification. Provided the correct classification rate using the test exceeds the correct classification rate under the null assumption, then the test is informative and may be clinically useful.

Even more important than correctly classifying all patients is the "positive predictive value" (PPV) of the test, which is equal to the number of true fast responders divided by the number of predicted fast responders. This is because the test will determine whether an intervention (i.e. earlier cessation of drug therapy) will be applied to fast responders. PPV is a function of sensitivity, specificity and prevalence, where:

$$PPV = (\text{sensitivity x prevalence}) / ((\text{sensitivity x prevalence}) + (1\text{-specificity}) \text{ x } (1\text{-prevalence}))$$

PPV >75% may be clinically useful, but PPV >90% would be much better. In the training set, PPV for classification of all patients was 9/10 = 90%. In the training set, PPV for classification of patients with any degree of fibrosis was 4/4 = 100%. In the prospective set, PPV for classification of all patients was 1/1 = 100%.

According to the present invention, the term "blood sample" refers particularly to a peripheral blood sample, particularly to whole blood samples treated with EDTA as an anticoagulant.

According to the present invention, the term "administering" refers to the administration of a therapeutically effective compound by any route, e.g. orally, parenterally (e.g., intramuscularly, intradermally, subcutaneously, etc.). The oral administration of the DAA compounds or compositions is subject-matter of one particular embodiment of the invention.

According to the present invention, the term "immunological method" refers to any method for the detection of the phenotype of cells, in particular, the phenotype of T cells that is sufficiently sensitive and specific for the detection of said phenotype, e.g. fluorescence-based methods such as flow cytometry, microscopy-based methods and the like.

According to the present invention, the term "molecular biological" refers to any method for the detection of the phenotype of cells, in particular, the phenotype of T cells that is sufficiently sensitive and specific for the detection of said phenotype using nucleic acid molecules of such cells, e.g. DNA and RNA. Examples for such molecular biological methods are all types of PCR (e.g. quantitative real-time PCR, Reverse-Transcription PCR, digital PCR, etc.) or other methods based on a sufficiently sensitive and specific hybridization of detectably labelled probes with nucleic acids that are indicative of a the presence and absence of a phenotype of interest and that preferably permit their quantification, e.g. Northern Blotting, FISH, etc. as known to the person skilled in the art.

According to the present invention, the term "CD" (for "Cluster of Differentiation") refers to a classification system for molecules found on cell surfaces. "CD3" stands for a T cell co-receptor that plays a role in the activation. "CCR7" is an abbreviation for the C-C Chemokine Receptor type 7 also known as CD 197. "CD45RA" is the RA isotype of the protein tyrosine phosphatase receptor, type C. "CD8" is a co-receptor of the T cell receptor and is often, but not exclusively, found on cytotoxic T cells.

As used herein, the term "frequency" in connection with a given CD molecule indicates the percentage amount of cells in a blood sample that have the respective phenotype amongst all leucocytes present in said sample. The absolute number of a specified subset of leucocytes is related to the absolute number of all leucocytes by its frequency within the pool of all leucocytes. Alternatively, "frequency" may designate percentage amount of cells in a blood sample that have the respective phenotype amongst a 'parent' or 'denominator' population of leucocytes of a specified phenotype that are present in said sample.

As used herein the expression "response rate" indicates that a desired reaction to a drug or combination of drugs is achieved or is not achieved in the individual/patient that was treated within a particular period of time. In the present case, the response rate may indicate whether or not the administered DAA compound or DAA composition was effective to result in HCV RNA negativity by 4 weeks (fast response rate) or later (slow response rate).

In one embodiment, the invention relates to a method for predicting a time-to-viral clearance status (i.e. using a PCR-based assay for viral load is considered "clinically clear" when the copy number of viral RNA per milliliter blood is <12; Virus may indeed be detectable, but it is below the level of a clinical infection) in a patient with chronic Hepatitis C Virus (HCV) infection under direct acting antiviral (DAA) therapy comprising the following steps:

a) Measuring the frequency and/or absolute number of CD3$^+$ T cells in a blood sample obtained from said individual prior to DAA therapy, and/or
b) Measuring the frequency and/or absolute number of naive CD8$^+$ T cells in said blood sample prior to DAA therapy,
c) Performing a logistic regression analysis of the cell frequencies and/or absolute numbers obtained in a) and b) to calculate the Odds ratios,
d) Based on step c), calculating a probability of fast- and/or slow-responder status in terms of time-to-viral clearance under DAA therapy,
e) Assigning a cut-off threshold according to test sensitivity and/or test specificity for the probabilities in step d),
f) Assigning the patient to a respective status.

In another embodiment, the present invention relates to the method of the preceding embodiment, wherein the naive CD8+ T cells are CCR7+ CD45RA+ naïve CD8+ T cells. Instead of using CCR7 is also possible to measure the expression of CD62L.

In another embodiment, the present invention relates to the methods of any one of the preceding embodiments, wherein a patient is classified as a slow responder in terms of time-to-viral clearance when the frequency and/or absolute number of CD3$^+$ T cells are above said cut-off threshold and/or when the frequency and/or absolute number of naïve CD8$^+$ T cells are below said cut-off threshold.

In another embodiment, the present invention relates to the methods of any one of the preceding embodiments, wherein a patient is classified as a fast responder in terms of time-to-viral clearance when the frequency and/or absolute number of CD3$^+$ T cells are below said cut-off threshold and/or when the frequency and/or absolute number of naïve CD8$^+$ T cells are above said cut-off threshold.

In other embodiments, the present invention relates to a method of any one of the preceding embodiments, further comprising at least one or more of the following steps:

g) Measuring the frequency and/or absolute number of CCR7$^-$ CD45RA- CD8$^+$ effector memory T cells in said blood sample prior to DAA therapy, and/or
h) Measuring the frequency and/or absolute number of CD27$^-$ CD57$^+$ CD8$^+$ chronically activated T cells in said blood sample prior to DAA therapy, and/or
i) Measuring the frequency and/or absolute number of CD5$^{-/low}$ CD8$^+$ T cells in said blood sample prior to DAA therapy, and/or

j) Measuring the level or variation in CD5 expression by CD8⁺ T cells in said blood sample prior to DAA therapy, and

k) Performing steps c) to f) according to the preceding embodiments for said at least one or more measurements obtained in steps g) to j).

In another embodiment, the present invention relates to the method of the preceding embodiments, optionally further comprising at least one or more of the following steps:

- Estimating the frequencies and/or absolute number of said T cell populations using alternative marker combinations as known to a person skilled in the art (e.g. as shown in the figures, for example, in Fig. 2) in said blood sample prior to DAA therapy, and/or
- Estimating the frequencies and/or absolute number of other peripheral blood leucocyte populations affected by the same pathological process that leads to informative changes in said leucocyte populations of a-g in said blood sample prior to DAA therapy as known to a person skilled in the art (e.g. as shown in the figures, for example, in Fig. 2), and
- Calculating a probability of treatment failure under DAA therapy, probability of clinical complications under DAA therapy or risk of clinical sequelae of HCV infection using said variables and/or
- Asserting reference ranges for said variables or values derived from said variables in order to obtain said clinical information in steps i) and j) referred to above, and
- Using predictions obtained by said method to inform management of patients with chronic HCV infection, not limited to determining duration of DAA therapy, selection of DAA regimen, scheduling of other investigations, and/or choice of co-treatments and patient counseling.

In another embodiment, the present invention relates to the method of the preceding embodiments, wherein a slow response rate is defined as failure to achieve HCV RNA negativity and/or a sustained virological response (SVR) within 2 days, 3 days, 4 days, 5 days, 6 days, 1 week, 2 weeks, 3 weeks or 4 weeks under DAA treatment.

In another embodiment, the present invention relates to the method of the preceding embodiments, wherein a fast response rate to said DAA therapy is defined as achieving HCV RNA negativity and/or a sustained virological response (SVR) within 2 days, 3 days, 4 days, 5 days, 6 days, 1 week, 2 weeks, 3 weeks or 4 weeks under DAA treatment

In another embodiment, the present invention relates to the method of the preceding embodiments, wherein the frequency of CD3⁺ T cells and/or naïve CD8⁺ is measured by means of an immunological method or a molecular biological method.

In another embodiment, the present invention relates to the method of the preceding embodiments, wherein the frequency and/or absolute number of cell types referred to in the preceding claims is measured by means of an immunological method, wherein said method is selected from the group comprising flow cytometry or immunofluorescence microscopy.

In another embodiment, the present invention relates to the method of the preceding embodiments, wherein the frequency and/or absolute number of cell types referred to in any of the preceding embodiments is inferred from an analyte or analytes selectively expressed by said cell types as measured by a method selected from the group comprising enzyme-linked immunosorptive assay (ELISA), bead array, ELISPOT, turbidimetry, RIA, CLIA, end-point PCR, quantitative PCR, RNA hybridization and/or bioassays. These methods are well-known to a person skilled in the art and are subject to various standard textbooks.

In another embodiment, the present invention relates to a method of stratifying a patient who is chronically infected with HCV for a suitable treatment option with a DAA, comprising performing the steps of any of the methods of the foregoing embodiments, wherein a patient who is identified as slow responder is assigned to a DAA treatment regimen lasting at least 6 weeks, or at least 8 weeks, or at least 10 weeks, or more preferably at least 12 weeks, and wherein a patient who is identified as fast responder is selected for a DAA treatment for less than 12 weeks, or less than 8 weeks, or less than 4 weeks, or more preferably less than 3 weeks, or most preferably less than 2 weeks.

In another embodiment, the present invention relates to the preceding embodiment, wherein a patient who is identified as slow responder is selected for a DAA treatment for at least 12 weeks, and/or wherein a patient that is identified as fast responder is selected for a DAA treatment for 8, 6, or 4 weeks, or more preferably less than 3 weeks, e.g., for 2 weeks, or most preferably for less than 2 weeks.

In another embodiment, the present invention relates to the preceding embodiments, wherein a patient that is identified as slow responder is selected for a DAA treatment for at least 24 weeks.

In another embodiment, the present invention relates to any of the preceding embodiments, wherein the DAA is selected from the group comprising NS3/4 protease inhibitors, NS5B nucleoside polymerase inhibitors, NS5B non-nucleoside polymerase inhibitors, and NS5A inhibitors.

For instance, a fast responder may be treated with the combination of ledipasvir and sofosbuvir, whereas a slow responder may be additionally treated with ribavarin. Alternatively, a fast responder may be treated with ritonavir-boosted paritaprevir, ombitasvir and dasabuvir, whereas a slow responder may be additionally treated with ribavarin. Alternatively, a fast responding patient may be treated with a short-course of (i) sofosbuvir, ledipasvir plus asunaprevir, (ii) sofosbuvir, daclatasvir plus simeprevir, or (iii) sofosbuvir, daclatasvir plus asunaprevir, whereas a slow responding patient may be treated with these regimens for 3 weeks and then receive an additional 8 or 12 weeks treatment with sofosbuvir and ledipasvir.

In another embodiment, the present invention relates to any of the preceding embodiments, wherein a patient who is identified as a slow responder is assigned to treatment with ribavirin in addition to a DAA regimen selected from the group comprising NS3/4 protease inhibitors, NS5B nucleoside polymerase inhibitors, NS5B non-nucleoside polymerase inhibitors, and NS5A inhibitors.

In another embodiment, the present invention relates to any of the preceding embodiments, wherein NS3/4 protease inhibitor is selected from the group comprising paritaprevir, wherein the NS5B nucleoside polymerase inhibitor is selected from the group comprising sofosbuvir, wherein the NS5B non-nucleoside polymerase inhibitor is selected from the group comprising dasabuvir, and wherein the NS5A inhibitor is selected from the group comprising ledipasvir and daclatasvir.

In another embodiment, the present invention relates to a direct-acting antiviral compound as defined in the preceding claims or a direct-acting antiviral composition in (a) therapeutically effective amount(s) for use in the treatment of a patient with chronic HCV infection, comprising

- determining the frequency of the $CD3^+$ T cells in a blood sample of said patient, and/or
- determining the frequency of naive $CD8^+$ T cells in a said blood sample,
- optionally determining the frequency of additional cell populations in said blood sample as defined in any of the foregoing embodiments,

wherein said individual has a baseline mean HCV RNA titer in the range of at least $10^4$ to at least $10^7$ IU/ml or, preferably, $>10^7$ IU/ml prior to DAA therapy, and/or
wherein the individual has previously not been treated with a DAA-based therapy, and/or
wherein the individual has previously been treated with [PEGylated] interferon alpha and/or non-DAA virostatics, and
wherein said therapeutically effective amount of said DAA compound or DAA composition is for administration for lasting at least 6 weeks, or at least 8 weeks, or at least 10 weeks, or more preferably at least 12 weeks, or for at least 24 weeks, if the patient is a slow responder as determined with a method according to any one of the in the preceding embodiments.

In another embodiment, the present invention relates to a direct-acting antiviral compound as defined in the preceding claims or a direct-acting antiviral composition in therapeutically effective amount(s) for use in the treatment of a patient with chronic HCV infection, wherein said direct-acting antiviral composition is for administration for at least 2 weeks, at least 4 weeks, for at least 12 weeks, or for at least 24 weeks, if the patient is a slow responder as determined with a method according to any one of the in the preceding embodiments.

In another embodiment, the present invention relates to a direct-acting antiviral compound as defined in the preceding claims or a direct-acting antiviral composition in therapeutically effective amount(s) for use in the treatment of a patient with chronic HCV infection, comprising

- determining the frequency of the $CD3^+$ T cells in a blood sample, and/or
- determining the frequency of naïve $CD8^+$ T cells in a said blood sample,
- optionally determining the frequency of additional cell populations in said blood sample as defined in any of the foregoing embodiments, and

wherein said individual has a baseline mean HCV RNA titer in the range of at least $10^4$ to at least $10^7$ IU/ml or, preferably, $>10^7$ IU/ml prior to DAA therapy, and/or
wherein the individual has previously not been treated with a DAA-based therapy, and/or
wherein the individual has previously been treated with [PEGylated] interferon alpha and/or non-DAA virostatics, and
wherein said therapeutically effective amount of said DAA compound or DAA composition is for administration for 8, 6, or 4 weeks, or more preferably less than 3 weeks, e.g., for 2 weeks, or most preferably for less than 2 weeks, if the patient is a fast responder as determined with a method according to any one of the in the preceding embodiments.

In further embodiments of the invention, the methods of predicting slow or fast response time-to-viral clearance in patients with chronic HCV infection that are or will be treated with DAA, and the methods of stratifying patients for a suitable treatment as discussed above according to the classifications as slow responder or fast responder to DAA are based on the following steps:

a) studying the markers indicative of respective sub-populations of lymphocytes as disclosed in the preceding sections by measuring their frequencies and/or absolute numbers to obtain respective numeric values;

b) combining said values through a logistic function including said markers in order to obtain an end value, wherein said logistic function is obtained through the following method:

i) classification of a cohort of patients in different groups according to the extent of their values measured in a);

ii) identification of factors which differ significantly between these groups by univariate analysis;

iii) logistic regression analysis to assess the independent discriminative value of markers indicative of the time-to-viral clearance;

iv) construction of the logistic function by combination of these identified independent markers; and

c) analyzing said end value of said logistic function in order to determine whether said patient is a fast responder or a slow responder to DAA treatment.

The invention also relates to methods of predicting slow or fast response time-to-viral clearance in patients with chronic HCV infection that are or will be treated with DAA, and the methods of stratifying patients for a suitable treatment as discussed above according to the classifications as slow responder or fast responder to DAA comprising measurement of the frequencies of expression of at least two, at least three, at least four, or more of the markers or combinations thereof indicative of respective sub-populations of lymphocytes set forth above, where the markers permit determining whether an individual is either a slow responder or a fast responder to DAA treatment as determined using a ROC curve analysis. Preferably, the Area Under the ROC curve is greater than 0.5, preferably greater than about 0.6, 0.7, 0.8, or greater than about 0.9.

As used herein "predicting time-to-viral clearance" according to the invention refers to a method of determining if a patient is a slow responder or a fast responder to a treatment with DAA. Therefore, in embodiments of the invention, "predicting time-to-viral clearance" refers to a statistical determination, with a sufficient degree of certainty, as between two options, namely that a patient is a slow responder or a fast responder to a treatment with DAA. As would be understood by a person skilled in the art, in this context a "sufficient degree of certainty" depends upon the sensitivity and specificity required for the prediction of time-to-viral clearance. More particularly the sufficient degree of certainty includes greater than 50% sensitivity and/or specificity, greater than 60% sensitivity and/or specificity, greater than 70% sensitivity and/or specificity, greater than 80% sensitivity and/or specificity, greater than 90% sensitivity and/or specificity and 100% sensitivity and/or specificity.

As used herein, "frequencies and/or absolute numbers of sub-populations of lymphocytes that may be characterized by the herein disclosed surface markers and that are indicative of a fast or slow response to DAA" means that the expression pattern is found significantly more often in patients with either a slow response or a fast response to DAA as disclosed herein. The respective frequencies and/or absolute numbers are then further analyzed using routine statistical methods setting confidence levels at a minimum of 70%, 75%, 80%, 85%, 90%, 95% and the like). Preferably, an expression pattern which is indicative of slow response to DAA is found in at least 70%, at least 75%, at least 80%, at least 85%, at least 90%, at least 95% or more in patients who actually show a slow response to DAA, and is found in less than 10%, less than 8%, less than 5%, less than 2.5%, or less than 1% of patients who do not show a slow response to DAA, and of course an expression pattern which is indicative of fast response to DAA is found in at least 70%, at least 75%, at least 80%, at least 85%, at least 90%, at least 95% or more in patients who actually show a fast response to DAA, and is found in less than 10%, less than 8%, less than 5%, less than 2.5%, or less than 1% of patients who do not show a fast response to DAA. The statistical methods of predicting the time-to-viral clearance or stratifying the patients into the above mentioned categories are made by logistic regression analysis, which may be performed by suitable computer software. The classification of the respective patients into the two categories can involve the use of cut-off thresholds or cut-off levels that are adapted for each specific subpopulation of lymphocytes characterized by the herein disclosed cell surface markers and are influenced by the sensitivity and/or specificity of the method of the respective method or assay that is used to measure these markers. Of course, it is possible to calculate standard deviations, e.g. the number of standard deviations may be two standard deviations.

Experiments

Clinical study design and Summary

Peripheral blood samples were provided by patients with chronic HCV infection, who were participating in an observational trial (clinicaltrials.gov: NCT02904603) authorized by the local ethics committee. For a training trial, 23 patients (genotype 1a (n=10), 1b (n=9) and 3 (n=4)) were treated with daclatasvir plus sofosbuvir (DCV/SOF; n=15), ledipasvir plus sofosbuvir (LDV/SOF; n=4) or with ritonavir-boosted paritaprevir, ombitasvir, and dasabuvir (n=4). Patient details are shown in Table 1, infra.

Blood sampling

Whole blood samples were collected in EDTA tubes by peripheral venepuncture and then immediately delivered to the laboratory at ambient temperature. Pre-analytical samples were stored at 4°C until processing began within 4 hours of sample collection. Whole blood was stained with ONE Study DuraClone panels (Beckman Coulter, Krefeld, Germany) according to the manufacturer's recommendations. Data was collected with a 10-colour Navios cytometer running Navios Clinical Software (Beckman Coulter). Analyses were performed using Kaluza version 1.3 (Beckman Coulter) by two independent operators blinded to clinical outcomes.

Statistics

Principal component analysis, receiver operator characteristic (ROC) curve analysis and binary logistic regression were performed in SPSS® release 23.0.0.0 (IBM Analytics, New York, USA). GraphPad Prism 6.04 (GraphPad Software, Inc., La Jolla, USA) was used for significance tests and generating plots.

Table 1 - Patient characteristics

| Patient | Age, y | Sex | HCV genotype | HCV-RNA $\log_{10}$ IU/ml | Fibrosis* | ALT level, U/L | Previous HCV treatment | DAA Therapy | Response to DAA Therapy |
|---|---|---|---|---|---|---|---|---|---|
| 1 | 62 | F | 1a | 7.2 | F1 | 88 | PegIFN/RBV/Teleprevir | SOF+DCV | SVR12[b] |
| 2 | 63 | M | 1a | 6.3 | F1 | 40 | none | SOF+DCV | SVR12 |
| 3 | 38 | M | 1a | 6.8 | F3 | 240 | PegIFN/RBV/Teleprevir | SOF+DCV | SVR12 |
| 4 | 69 | M | 1b | 7.0 | F4 | 134 | PegIFN/RBV/Boceprevir | SOF+DCV | SVR12 |
| 5 | 47 | M | 1a | 6.1 | F4 | 103 | PegIFN/RBV | SOF+DCV® | SVR12 |
| 6 | 61 | M | 1b | 6.5 | F3 | 117 | none | SOF+DCV | SVR12 |
| 7 | 46 | M | 1b | 5.2 | F4 | 208 | none | SOF+DCV | SVR12 |
| 8 | 58 | M | 1a | 6.1 | F0 | 119 | PegIFN/RBV | SOF+DCV | SVR12 |
| 9 | 62 | M | 1a | 6.2 | F4 | 142 | PegIFN/RBV | SOF+DCV® | SVR12 |
| 10 | 55 | M | 1a | 5.5 | F4 | 140 | none | SOF+DCV | SVR12 |
| 11 | 77 | F | 1b | 5.6 | F0 | 45 | none | SOF+DCV | SVR12 |
| 12 | 64 | M | 3a | 5.0 | F2 | 165 | none | SOF+DCV | SVR12 |
| 13 | 56 | M | 3a | 6.4 | F1 | 44 | PegIFN/RBV | SOF+DCV | SVR12 |
| 14 | 30 | M | 3a | 6.0 | F0 | 161 | none | SOF+DCV | relapse® |
| 15 | 68 | M | 1b | 6.6 | F0 | 81 | PegIFN/RBV | SOF+LDV | SVR12 |
| 16 | 59 | M | 1a | 6.3 | F0 | 103 | PegIFN/RBV | SOF+LDV | SVR12 |
| 17 | 57 | F | 1b | 6.0 | F0 | 55 | none | 30 | SVR12 |
| 18 | 57 | M | 1a | 5.3 | F0 | 32 | none | SOF+LDV® | SVR12 |
| 19 | 41 | F | 1b | 5.6 | F0 | 30 | PegIFN/RBV | 3D | SVR12 |
| 20 | 50 | M | 1b | 6.5 | F1 | 49 | PegIFN/RBV | 3D | SVR12 |
| 21 | 24 | F | 3a | 5.8 | F4 | 207 | none | SOF+DCV | SVR12 |
| 22 | 48 | M | 1a | 6.7 | F3 | 66 | none | 3D+RBV | SVR12 |

(continued)

| Patient | Age, y | Sex | HCV genotype | HCV-RNA log$_{10}$ IU/ml | Fibrosis* | ALT level, U/L | Previous HCV treatment | DAA Therapy | Response to DAA Therapy |
|---|---|---|---|---|---|---|---|---|---|
| 23 | 77 | F | 1b | 5.0 | F2 | 78 | none | SOF+LDV[e] | SVR12 |

Footnotes to Table 1:

ALT, alanine aminotransferase SOF, sofosbuvir DCV, daclatasvir LDV, ledipasvir 3D, paritaprevir (PTV) boosted by ritonavir (PTV/r) coformulated with ombitasvir (OBV) and given with dasabuvir (DSV) RVB, ribavirin

[a]determined by Acoustic Radiation Force Impulse (ARFI) Imaging; Colombo S. et al. J Gastroenterol (2012) 47: 461-469

[b]SVR12, sustained virological response at week 12 after end of treatment (ie, undetectable HCV-RNA)

[c]relaps, undetectable HCV-RNA at end of treatment (EOT) but detectable HCV RNA during follow-up

[d]treatment extended to 24 weeks

[e]treatment shortened to 8 weeks

Immunological markers affected by DAA treatment.

A sustained virological response to DAA therapy is generally achieved in 85-99% of chronic HCV patients, depending upon viral genotype, patient-related factors and the choice of treatment regime. In the present study of 23 patients with chronic HCV infection, 22 had achieved a sustained virological response at 12 weeks post-treatment (SVR12). Clearance of virus was associated with improved liver function and reduction in systemic markers of liver inflammation. Although most patients achieved sustained virological responses by 12 weeks, there was considerable variation in time to HCV RNA negativity and normalization of biochemical parameters. Baseline mean HCV RNA titer was $6.12\pm0.12$ log10IU/ml, falling to $1.2\pm0.14$ log10IU/ml by 4 weeks, at which time 12/23 patients were HCV RNA <12 IU/ml. HCV establishes persistent infections during which virus-specific CD4+ and CD8+ T cells become impaired through progressive functional exhaustion or clonal deletion, and intrahepatic NK cell responses are subdued. Hence, the objective of this study was to explain variation in 4-week response rates to DAA therapy in terms of immunological factors inherent to patients before treatment. In particular, this study aimed to identify a minimal set of immunological markers that accurately classified patients with chronic HCV infections as "fast responders" (i.e. HCV RNA <12 IU/ml by 4 weeks) or "slow responders" (i.e. HCV RNA $\geq$12 IU/ml at 4 weeks, irrespective of later outcome). These biomarkers were to be independent of viral genotype, baseline fibrosis score, previous HCV treatment, and other patient-related factors. Therefore, the influence of the ALT levels, of the HCV genotype, of a previous treatment for chronic HCV, and the pre-treatment fibrosis score were also analyzed.

Pre-treatment ALT levels

Baseline ALT levels were not associated with fast or slow responses.

|  |  | ALT level (U/L) | | |
|---|---|---|---|---|
|  |  | <50 | 50-150 | >150 |
| Response | Fast | 4 | 6 | 2 |
|  | Slow | 2 | 6 | 3 |

$\chi^2$ test: p = 0.662

HCV Genotype

HCV genotype was not associated with fast or slow responses.

|  |  | HCV Genotype | | |
|---|---|---|---|---|
|  |  | 1a | 1b | 3a |
| Response | Fast | 5 | 4 | 3 |
|  | Slow | 5 | 5 | 1 |

$\chi^2$ test: p = 0.586

Previous treatment for chronic HCV

Patients were categorised as having previously been treated with PegIFN/RBV-based therapies or not. Previous treatment was not significantly associated with fast or slow responses to DAA treatment.

|  |  | Previous Treatment | |
|---|---|---|---|
|  |  | Yes | No |
| Observed | Fast | 7 | 5 |
|  | Slow | 4 | 7 |

$\chi^2$ test: p = 0.292

Pre-treatment fibrosis score

Absence of liver fibrosis at baseline was not associated with fast or slow responses.

| | | Fibrosis Score | | | | |
|---|---|---|---|---|---|---|
| | | F0 | F1 | F2 | F3 | F4 |
| Response | Fast | 6 | 2 | 1 | 1 | 2 |
| | Slow | 2 | 2 | 1 | 2 | 4 |

$\chi^2$ test: p = 0.564

To further test the capacity of baseline fibrosis score to correctly classify patients as fast or slow responders, the table was collapsed as follows:

Grouping 1:

| | | Predicted | |
|---|---|---|---|
| | | Fast (F0) | Slow (F1 - F4) |
| Observed | Fast | 6 | 6 |
| | Slow | 2 | 9 |

$\chi^2$ test: p = 0.110; correct classification rate = 65.2%

Grouping 2:

| | | Predicted | |
|---|---|---|---|
| | | Fast (F0 - F1) | Slow (F2 - F4) |
| Observed | Fast | 8 | 4 |
| | Slow | 4 | 7 |

$\chi^2$ test: p = 0.146; correct classification rate = 65.2%
These data show that absence of fibrosis (or low-grade fibrosis) is not associated with being a fast responder.

Flow cytometry

Flow cytometry is a reliable and cost-effective platform for measuring immunological markers in peripheral blood. Following standardized flow cytometry protocols developed by The ONE Study consortium (www.onestudy.com) frequencies and absolute counts of 79 predefined leucocyte subsets in serial whole blood samples were measured. Baseline samples were taken immediately before DAA treatment started, followed by sampling at 4, 12 and 24 weeks after start of therapy. This immunophenotyping strategy gave a high-resolution picture of all major leucocyte subsets in blood, including their activation or maturation status. A blinded operator evaluated all flow cytometry data at one time and analyses were checked by a second blinded operator. From these results, information about 79 parameters from 23 patients sampled at 4 visits was assembled into a single dataset.
It was analyzed whether the baseline data contained the information to distinguish fast and slow responders. By standardizing the data and reducing their complexity by principal component analysis (PCA), partial separation of fast- and slow-responding patients was possible (Fig.1A).
Subsequently, it was determined whether a set of baseline immunological parameters could be identified that distinguished patients as fast- or slow-responders. To restrict the number of independent variables in our models, only parameters that changed significantly between pre-and post-treatment samples were considered. This approach was based on the assumption that changes in immunological parameters after DAA treatment were likely to reflect changes in the immune response against the virus or reflected indirect effects of changes in anti-viral immunity.
Changes in baseline-subtracted leucocyte subset frequencies between visits were identified by pairwise significance testing. After starting DAA treatment, an increase was observed in the frequency of CD8+ T cells, and CD4+ and CD8+ central memory T cells (TCM); by contrast, a decrease was observed in the frequency of CD4+ and CD8+ effector memory T cells (TEM), CD4+ TEMRA cells, naïve CD4+ T cells, non-classical CD14+CD16+ monocytes and CD56bright NK cells. Filtering the dataset for only these significantly changed variables improved separation of fast and slow responders by PCA (Fig.1B).

Model to predict fast and slow responses

Univariate analyses were performed to determine which of those parameters affected by DAA treatment were most significantly associated with fast or slow responder status (Fig.1C-E). As significant, robust and non-redundant marker populations, CD3+ T cells and CCR7+ CD45RA+ naïve CD8+ T cell frequencies were entered as independent variables into a binary logistic regression model. Receiver operator characteristic (ROC) curve analysis (Area=0.909; S.E.=0.61) was used to determine a cut-off value of the predicted probabilities that maximized both sensitivity (75.0%) and specificity (91.0%) (Fig.1C).

Table 2: *Logistic regression analysis of baseline leucocyte frequencies.* **(A)** Odds ratios and 95% confidence intervals. **(B)** Classification of 23 study patients as either fast or slow responders to DAA treatment according to only baseline CD3+ T cell and CCR7+ CD45RA+ naive CD8+ T cell frequencies. **(C)** Classification of 15 study patients with hepatic fibrosis as either fast or slow responders to DAA treatment according to only baseline CD3+ T cell and CCR7+ CD45RA+ naïve CD8+ T cell frequencies. **(D)** Prospective classification of 10 patients.

Table 2A.

| Parameter | Odds ratio (95%-CI) | p-value |
|---|---|---|
| CCR7+ CD45RA+ naïve CD8+ T cells | 1.126 (1.000, 1.269) | .051 |
| CD3+ T cells | 0.862 (0.729, 1.021) | .087 |

Table 2B.

| Classification Table*: All Patients | | | |
|---|---|---|---|
| | | Observed | |
| Predicted | | Response | Percentage |
| | | Slow | Fast | |
| Response | Slow | 10 | 3 | 76.9 |
| | Fast | 1 | 9 | 90.0 |
| Overall percentage | | | | 82.6 |
| *cut-off value is 0.66 | | | | |

Table 2C.

| Classification Table: Patients with Fibrosis Score 1-4 | | | |
|---|---|---|---|
| | | Observed | |
| Predicted | | Response | Percentage |
| | | Slow | Fast | |
| Response | Slow | 9 | 2 | 81.8 |
| | Fast | 0 | 4 | 100.0 |
| Overall percentage | | | | 86.7 |
| *cut-off value is 0.66 | | | | |

Table 2D.

| Classification Table*: Prospective Set | | | | |
|---|---|---|---|---|
| Predicted | | Observed | | |
| | | Response | | Percentage |
| | | Slow | Fast | |
| Response | Slow | 8 | 1 | 88.9 |
| | Fast | 0 | 1 | 100.0 |
| Overall percentage | | | | 90.0 |
| *cut-off value is 0.66 | | | | |

Baseline score for liver fibrosis was not associated with responder status. Therefore, it was checked if immunological markers classified the 15/23 study patients with any degree of liver fibrosis (Table 2C). The overall correct classification rate was 86.6%, compared to 60.0% correct classification under the assumption that all patients with liver fibrosis were to be slow responders. Importantly, the true positive rate for fast responders with liver fibrosis was 4/4 (100%) patients; accordingly, the herein disclosed methods will be particularly useful in identifying which patients with chronic HCV infection and liver fibrosis will be fast responders to DAA treatment. In particular, the herein disclosed methods will help predicting which patients with chronic HCV infections on the wait-list for liver transplantation are likely to achieve complete clearance of virus after DAA treatment within a typical waiting time.

Table 3 Patient outcomes

| Patient | HCV-RNA IU/ml | | | | ALT level, U/L | | | |
|---|---|---|---|---|---|---|---|---|
| | wk0 | wk4 | EOT[a] | SVR12[b] | wk0 | wk4 | EOT[a] | SVR12[b] |
| 1 | 1,5 x 10^7 | 1,3 x 10^2 | <12 | neg. | 88 | 29 | 22 | 25 |
| 2 | 1,9 x 10^6 | 1,6 x 10^1 | <12 | neg. | 40 | 24 | 23 | 21 |
| 3 | 6,5 x 10^6 | 5,2 x 10^2 | <12 | neg. | 240 | 23 | 19 | 23 |
| 4 | 1,0 x 10^7 | 4,2 x 10^1 | <12 | neg. | 134 | 34 | 31 | 42 |
| 5 | 1,2 x 10^6 | <12 | neg. | neg. | 103 | 38 | 44 | 31 |
| 6 | 3,1 x 10^6 | 1,3 x 10^2 | <12 | neg. | 117 | 42 | 46 | 35 |
| 7 | 1,6 x 10^5 | 1,5 x 10^1 | <12 | neg. | 208 | 27 | 24 | 21 |
| 8 | 1,4 x 10^6 | <12 | neg. | neg. | 119 | 51 | 45 | 41 |
| 9 | 1,6 x 10^6 | 4,0 x 10^1 | <12 | neg. | 142 | 47 | 61 | 37 |
| 10 | 3,1 x 10^5 | 3,6 x 10^1 | neg. | neg. | 140 | 34 | 42 | 43 |
| 11 | 3,9 x 10^5 | 5,7 x 10^1 | neg. | neg. | 45 | 25 | 30 | 24 |
| 12 | 1,1 x 10^5 | 4,8 x 10^1 | <12 | neg. | 165 | 62 | 70 | 62 |
| 13 | 2,3 x 10^6 | neg | neg. | neg. | 44 | 22 | 17 | 17 |
| 14 | 1,1 x 10^6 | <12 | neg. | 1,6 x 10^4 | 161 | 31 | 23 | 107 |
| 15 | 3,6 x 10^6 | <12 | neg. | neg. | 81 | 31 | 31 | 27 |
| 16 | 2,1 x 10^6 | <12 | neg. | neg. | 103 | 74 | 64 | 32 |
| 17 | 1,1 x 10^6 | 1,5 x 10^1 | neg. | neg. | 55 | 24 | 21 | 24 |
| 18 | 2,2 x 10^5 | <12 | neg. | neg. | 32 | 22 | 23 | 21 |
| 19 | 4,1 x 10^5 | neg | neg. | neg. | 30 | 17 | 15 | 21 |
| 20 | 3,0 x 10^6 | neg | neg. | neg. | 49 | 19 | 23 | n.t. |
| 21 | 6,1 x 10^5 | <12 | neg. | neg. | 207 | 28 | 47 | 21 |
| 22 | 4,6 x 10^6 | <12 | neg. | neg. | 66 | 49 | 46 | 16 |
| 23 | 1,1 x 10^5 | <12 | neg. | neg. | 78 | 24 | 24 | 10 |

Footnotes to Table 3:

ALT, alanine aminotransferase

[a]EOT, end of treatment

[b]SVR12, sustained virological response at week 12 after end of treatment (ie, undetectable HCV-RNA)

To verify that the model accurately predicted fast and slow responses, data were collected from a further 10 chronic HCV patients prior to DAA treatment. In this group, 8 patients were slow responders and 2 were fast responders: all slow responders were correctly classified as such (Table 2D). The model also correctly identified 1 of 2 fast responders. Importantly, the only patient predicted to be a fast responder proved to be so. For the purpose of prospectively and safely shortening DAA therapy, a test with high specificity is more important than one with high sensitivity.

Mechanisms behind the predictive model

The question why a predictive model based on memory T cell subset distribution should correctly classify fast or slow responders to DAA therapy when the drugs' action does not apparently rely on immunological mechanisms was analyzed. To this end, combined results from the 'training' and 'prospective' datasets were analyzed. Because slow responders were characterized by higher frequencies of $CD3^+$ T cells and $CD8^+$ $T_{EM}$ cells, as well as lower frequencies of naïve $CD8^+$ T cells, a reanalysis focused on markers of $CD8^+$ T cell activation and differentiation. The most prominent finding was an over-representation of $CD27^-$, $CD57^+$ and $CD27^-$ $CD57^+$ $CD8^+$ T cells in slow responders (Fig.2A-C), implying a relative accumulation of chronically activated $CD8^+$ T cells in those individuals. These immunological parameters in the predictive model of the invention are surrogate markers of an HCV-specific response, which would otherwise be extremely difficult to measure with routine clinical diagnostic assays. There was also a tendency for $CD8^+$ T cells from fast-responders to express lower levels of CD5 than $CD8^+$ T cells from slow-responders (Fig.2D&E). Significantly, fast-responders exhibited a broader spread of CD5 expression in $CD8^+$ T cells than slow-responders (Fig.2D&F). CD5 down-regulation was confined to $CD27^-$ $CD8^+$ T cells (Fig.2G).

A consolidated assay

In order to simplify and standardize the analytical methods, a single, 10-colour flow cytometry panel to measure frequencies of $CD3^+$ T cells, $CD45RA^+$ $CCR7^+$ $CD8^+$ naïve T cells, $CD27^-$ $CD57^+$ $CD8^+$ T cells and CD5-expressing $CD8^+$ T cells (Table 2) was created. One channel is available for live/dead discrimination using 7-AAD, meaning that this panel can be used for fresh or stored material. Absolute quantification of CD5 expression is possible using reference material. All antibodies can be obtained from a single supplier in a premixed, dried-down format, which can help to reduce technical variation. Populations of interest are clearly resolved and gating is intuitive (Fig.3).

Table 4: A consolidated flow cytometry panel.

| Figure 4 - Consolidated flow cytometry panel and recommended gating strategy. Example data from one patient with chronic HCV infection is shown. | | | | | | | | | | |
|---|---|---|---|---|---|---|---|---|---|---|
| | 488 Excitation | | | | | 633 Excitation | | | 405 Excitation | |
| | FL1 (FITC) | FL2 (PE) | FL3 (ECD) | FL4 (7-AAD) | FL5 (PE-Cy7) | FL6 (APC) | FL7 (AF700) | FL8 (APC-AF750) | FL9 (PacBlue) | FL10 (Aqua) |
| ANTIGEN | CD45RA | CD5 | CD27 | Live-Dead | CCR7 | CD4 | CD8 | CD3 | CD57 | CD45 |
| Clone name | 2H4 | BL1a | 1A4CD27 | Optional | G043H7 | 13B8.2 | B9.11 | UCHT1 | NC1 | J.33 |
| Isotype | mIgG1 | mIgG2a | mIgG1 | | mIgG2a | mIgG1 | mIgG1 | mIgG1 | mIgM | mIgG1 |
| Amount (µl) | 8 | 20 | 8 | + | 10 | 6 | 2 | 8 | 8 | 8 |
| Supplier | BC | BC | BC | BC | BC | BC | BC | BC | BC | BC |
| Cat. # | 6603904 | A07753 | B26603 | A07704 | B46025 | IM2468 | B49181 | A94680 | A74779 | B36294 |
| Status | RUO | CE | ASR | CE | ASR | CE | CE | CE | ASR | CE |

To verify that the herein disclosed model accurately predicts fast and slow responses, data were collected from a further 10 chronic HCV patients prior to DAA treatment (*cf.* the Table 5 below).

Table 5

| Patient | Age, y | Sex | HCV genotype | HCV-RNA $\log_{10}$ IU/ml | Fibrosis® | ALT level, U/L | Previous HCV treatment | DAA Therapy | Response to DAA Therapy wk4 IU/ml |
|---|---|---|---|---|---|---|---|---|---|
| 1 | 64 | F | 1a | 7.1 | F4 | 56 | IFN | SOF+LDV+RBV | 130 |
| 2 | 60 | M | 3a | 5.5 | n.t. | 149 | PegIFN/RBV, SOF/RBV | SOF+DCV | 30 |
| 3 | 51 | M | 2b | 5.7 | F1 | 54 | none | SOF+RBV | 17 |
| 4 | 72 | F | 1a | 5.9 | F1 | 44 | PegIFN/RBV | SOF+LDV | 12 |
| 5 | 72 | F | 1b | 5.9 | F1 | 43 | none | SOF+LDV | <12 |
| 6 | 59 | F | 1a | 6.2 | F1 | 69 | none | SOF+LDV | 61 |
| 7 | 43 | M | 1a | 5.6 | F1 | 80 | none | SOF+LDV | <12 |
| 8 | 63 | M | 1a | 6.1 | F2 | 39 | none | SOF+LDV | 51 |
| 9 | 26 | F | 1a | 6.3 | F1 | 23 | PegIFN/RBV | SOF+LDV | 12 |
| 10 | 53 | F | 1a | 6.5 | F1 | 54 | PegIFN/RBV | SOF+LDV | 14 |

Footnotes to Table 5:
ALT, alanine aminotransferase
RBV, ribavirin
DCV, daclatasvir
SOF, sofosbuvir
LDV, ledipasvir
[a]determined by Acoustic Radiation Force Impulse (ARFI) Imaging; Colombo S. et al. J Gastroenterol (2012) 47: 461-469

In this group, 8 patients were slow responders and 2 were fast responders: all slow responders were corrected classified as such (see above Table 1D). The model correctly identified 1 of 2 fast responders. Importantly, the only patient predicted to be a fast responder proved to be so. For the purpose of prospectively and safely shortening DAA therapy, a test with high specificity is more important than one with high sensitivity.

In order to find out, if a predictive model based on memory T cell subset distribution may correctly classify fast or slow responders to DAA therapy, combined results from the 'training' and 'prospective' datasets were analyzed. Because slow responders were characterized by higher frequencies of $CD3^+$ T cells and $CD8^+$ $T_{EM}$ cells, as well as lower frequencies of naive $CD8^+$ T cells, a re-analysis focused on markers of $CD8^+$ T cell activation and differentiation. The most prominent finding was an over-representation of $CD27^-$, $CD57^+$ and $CD27^-$ $CD57^+$ $CD8^+$ T cells in slow responders (Fig.2A-C), implying a relative accumulation of chronically activated $CD8^+$ T cells in those individuals.

These activated T cells are too frequent to be virus-specific; nevertheless, their relative abundance seems to be an indirect consequence of chronic HCV infection. It is known that HCV-specific, T cell-mediated responses enhance 'bystander' activation of virus-nonspecific T cells through release of inflammatory mediators, which systemically down-regulates CD5 expression by naïve $CD8^+$ T cells, lowering their activation threshold. Accordingly, it is considered immunological parameters used in a predictive model are surrogate markers of an HCV-specific response, which would otherwise be extremely difficult to measure with routine clinical diagnostic assays. The initial finding that slow responders exhibited higher frequencies of activated and memory phenotype $CD8^+$ T cells than fast responders seems to contradict this hypothesis. However, as the relationship between effectiveness or chronicity of specific HCV responses and the magnitude of bystander $CD8^+$ T cell activation is not understood the results presented herein might be explained by a greater or more chronic, but less effective virus-specific responses cumulating in greater bystander hyper-activation over time. In support of this explanation, $CD8^+$ T cells from slow-responders expressed higher levels of CD5 than $CD8^+$ T cells from fast-responders (Fig.2D). Baseline viral load was not different between fast and slow responders (Fig.2E). Furthermore, no correlation was observed between predictive scores and viral load at baseline (Fig.2F) or pre-treatment $CD27^-$ $CD57^+$ $CD8^+$ T cell frequency and viral load at baseline (Fig.2G).

The above experiment provide an unbiased approach to biomarker discovery by screening chronic HCV patients receiving DAA treatment for changes in diverse peripheral blood leucocyte populations, including subsets of T cells, B cells, NK cells, monocytes and blood dendritic cells (DC). Prominent, but focused changes in the frequencies of non-classical monocytes, $CD56^{bright}$ NK cells and memory T cell subsets were associated with viral clearance after DAA treatment; however, no definite effects were found in B cell, classical NK cell and blood DC subsets.

The relatively narrow immunological impact of DAA treatment is convenient when building a predictive model because fewer independent variables need to be considered. In this study, we found that knowing only pre-treatment frequencies of $CD3^+$ T cells and $CCR7^+$ $CD45RA^+$ naive $CD8^+$ T cells allowed 82.6% of patients to be correctly classified as fast or slow responders.

Advantageously, a true positive prediction rate of 90.0% means that the classifier performs surprisingly well in identifying patients as fast responders. A method of reliably identifying fast responders opens the possibility of treating them with shorter courses of DAA therapy, thereby cutting costs to healthcare providers. Current EASL guidelines recommend that a small subgroup of treatment-naive patients without cirrhosis and with GT1 infection can be treated for only 8 weeks if their baseline HCV RNA level is below 6 million IU/ml.(8) The herein provided embodiments can eventually be used to extend this recommendation to predicted fast-responders.

The features disclosed in the foregoing description, in the claims and/or in the accompanying drawings may, both separately and in any combination thereof, be material for realizing the invention in diverse forms thereof.

**Claims**

1. A method for predicting a time-to-viral clearance status in a patient with chronic Hepatitis C Virus (HCV) infection under direct acting antiviral (DAA) therapy comprising the following steps:

   a) Measuring the frequency and/or absolute number of $CD3^+$ T cells in a blood sample obtained from said individual prior to DAA therapy, and/or
   b) Measuring the frequency and/or absolute number of naïve $CD8^+$ T cells in said blood sample prior to DAA therapy,
   c) Performing a logistic regression analysis of the cell frequencies and/or absolute numbers obtained in a) and b) to calculate the Odds ratios,
   d) Based on step c), calculating a probability of fast- and/or slow-responder status in terms of time-to-viral clearance under DAA therapy,
   e) Assigning a cut-off threshold according to test sensitivity and/or test specificity for the probabilities in step d),
   f) Assigning the patient to a respective status.

2. The method of claim 1, wherein the naive CD8+ T cells are CCR7+ CD45RA+ naive CD8+ T cells.

3. The method of claim 1, wherein a patient is classified as a slow responder in terms of time-to-viral clearance when the frequency and/or absolute number of $CD3^+$ T cells and/or when the frequency and/or absolute number of naive $CD8^+$ T cells are used in a predictive model to calculate a probability of fast responder status below said cut-off threshold.

4. The method of claim 1, wherein a patient is classified as a fast responder in terms of time-to-viral clearance when the frequency and/or absolute number of $CD3^+$ T cells and/or when the frequency and/or absolute number of naïve $CD8^+$ T cells are used in a predictive model to calculate a probability of fast responder status above said cut-off threshold.

5. The method of any one of claims 1 to 4, optionally further comprising at least one or more of the following steps:

   g) Measuring the frequency and/or absolute number of $CCR7^-$ CD45RA- $CD8^+$ effector memory T cells in said blood sample prior to DAA therapy, and/or
   h) Measuring the frequency and/or absolute number of CD27- CD57+ CD8+ chronically activated T cells in said blood sample prior to DAA therapy, and/or
   i) Measuring the frequency and/or absolute number of CD5-/low CD8+ T cells in said blood sample prior to DAA therapy, and/or
   j) Measuring the level or dispersion of CD5 expression by CD8+ T cells in said blood sample prior to DAA therapy, and
   k) performing steps c) to f) according to the preceding claims for said at least one or more measurements obtained in steps g) to j).

6. The method according to any one of claims 1 to 5, wherein a slow response rate is defined as failure to achieve HCV RNA negativity and/or a sustained virological response (SVR) within 2 days, 3 days, 4 days, 5 days, 6 days, 1 week, 2 weeks, 3 weeks or 4 weeks under DAA treatment.

7. The method according to claim 1 to 6, wherein a fast response rate to said DAA therapy is defined as achieving HCV RNA negativity and/or a sustained virological response (SVR) within 2 days, 3 days, 4 days, 5 days, 6 days, 1 week, 2 weeks, 3 weeks or 4 weeks under DAA treatment.

8. The method according to any one of claims 1 to 7, wherein the frequency of $CD3^+$ T cells and/or naive $CD8^+$ is measured by means of an immunological method or a molecular biological method.

9. The method according to any one of the preceding claims, wherein the frequency and/or absolute number of informative cell types referred to in said claims is measured by means of an immunological method, wherein said method is selected from the group comprising flow cytometry or immunofluorescence microscopy.

10. The method according to any one of claims 1 to 9, wherein the frequency and/or absolute number of cell types referred to in any of the preceding claims is inferred from an analyte or analytes selectively expressed by said cell types as measured by a method selected from the group comprising enzyme-linked immunosorptive assay (ELISA), bead array, ELISPOT, turbidimetry, RIA, CLIA, end-point PCR, quantitative PCR, RNA hybridization and/or bioassays.

11. A method of stratifying a patient who is chronically infected with HCV for a suitable treatment option with a DAA, comprising performing the steps of any of the preceding methods of claims 1 to 10,
    wherein a patient who is identified as slow responder is assigned to a DAA treatment regimen lasting at least 6 weeks, or at least 8 weeks, or at least 10 weeks, or more preferably at least 12 weeks, and/or
    wherein a patient who is identified as fast responder is selected for a DAA treatment for less than 12 weeks, or less than 8 weeks, or less than 4 weeks, or more preferably less than 3 weeks, or most preferably less than 2 weeks.

12. The method according to claim 11, wherein a patient who is identified as slow responder is selected for a DAA treatment for at least 12 weeks, and/or
    wherein a patient that is identified as fast responder is selected for a DAA treatment for 8, 6, or 4 weeks, or more preferably less than 3 weeks, or most preferably less than 2 weeks

13. The method according to any one of claims 11 and 12, wherein a patient that is identified as slow responder is selected for a DAA treatment for at least 24 weeks.

14. The method of any one of the preceding claims, wherein the DAA is selected from the group comprising NS3/4 protease inhibitors, NS5B nucleoside polymerase inhibitors, NS5B non-nucleoside polymerase inhibitors, and NS5A inhibitors.

15. The method of any one of the preceding claims, wherein a patient who is identified as a slow responder is assigned to treatment with ribavirin in addition to a DAA regimen selected from the group comprising NS3/4 protease inhibitors, NS5B nucleoside polymerase inhibitors, NS5B non-nucleoside polymerase inhibitors, and NS5A inhibitors.

16. The method of any one of the preceding claims, wherein NS3/4 protease inhibitor is selected from the group comprising paritaprevir, wherein the NS5B nucleoside polymerase inhibitor is selected from the group comprising sofosbuvir, wherein the NS5B non-nucleoside polymerase inhibitor is selected from the group comprising dasabuvir, and wherein the NS5A inhibitor is selected from the group comprising ledipasvir and daclatasvir.

17. A direct-acting antiviral compound as defined in the preceding claims or a direct-acting antiviral composition in therapeutically effective amount(s) for use in the treatment of a patient with chronic HCV infection, comprising

   o determining the frequency of the $CD3^+$ T cells in a blood sample of said patient, and/or
   o determining the frequency of naïve $CD8^+$ T cells in a said blood sample,

   wherein said individual has a baseline mean HCV RNA titer in the range of at least $10^4$ to at least $10^7$ IU/ml or, preferably, >$10^7$ IU/ml prior to DAA therapy, and/or
   wherein the individual has previously not been treated with a DAA-based therapy, and/or
   wherein the individual has previously been treated with [PEGylated] interferon alpha and/or non-DAA virostatics, and wherein said therapeutically effective amount of said DAA compound or DAA composition is for administration for more than 8 weeks, for at least 12 weeks or for at least 24 weeks, if the patient is a slow responder as determined with a method according to any one of the preceding claims.

18. The direct-acting antiviral compound as defined in the preceding claims or a direct-acting antiviral composition in therapeutically effective amount(s) for use in the treatment of a patient with chronic hepatitis C virus infection according to claim 17, wherein said direct-acting antiviral composition is for administration for at least 2 weeks, at least 4 weeks, for at least 12 weeks, or for at least 24 weeks, if the patient is a slow responder as determined with a method according to any one of the preceding claims.

19. A direct-acting antiviral compound as defined in the preceding claims or a direct-acting antiviral composition in therapeutically effective amount(s) for use in the treatment of an individual with chronic hepatitis C virus infection, comprising

   ∘ determining the frequency of the $CD3^+$ T cells in a blood sample, and/or
   ∘ determining the frequency of naïve $CD8^+$ T cells in a said blood sample, and

   wherein said individual has a baseline mean HCV RNA titer in the range of at least $10^4$ to at least $10^7$ IU/ml or, preferably, > $10^7$ IU/ml prior to DAA therapy, and/or
   wherein the individual has previously not been treated with a DAA-based therapy, and/or
   wherein the individual has previously been treated with [PEGylated] interferon alpha and/or non-DAA virostatics, and wherein said therapeutically effective amount of said DAA compound or DAA composition is for administration for not more than for 4 weeks, if the patient is a fast responder as determined with a method according to any one of the preceding claims.

Figure 1

Figure 2

Figure 3

Figure 4

Europäisches
Patentamt

European
Patent Office

Office européen
des brevets

# EUROPEAN SEARCH REPORT

Application Number

EP 17 16 6600

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHAO ZHANG ET AL: "Comprehensive mapping of antigen specific T cell responses in hepatitis C virus infected patients with or without spontaneous viral clearance", PLOS ONE, vol. 12, no. 2, 7 February 2017 (2017-02-07), page e0171217, XP55388702, DOI: 10.1371/journal.pone.0171217 | 1-10, 17-19 | INV. G01N33/576 |
| Y | (p 2, para 2)(p 9, last para)(p 13, para 1)(Fig 4F); * abstract * | 11-16 | |
| X | M. A. BURCHILL ET AL: "Memory re-differentiation and reduced lymphocyte activation in chronic HCV-infected patients receiving direct-acting antivirals", JOURNAL OF VIRAL HEPATITIS., vol. 22, no. 12, 7 December 2015 (2015-12-07), pages 983-991, XP055388707, OXFORD, UK ISSN: 1352-0504, DOI: 10.1111/jvh.12465 * abstract; figure 1 * | 1-10 | TECHNICAL FIELDS SEARCHED (IPC) <br><br> G01N |
| X | KATE CHILDS ET AL: "Immunological Predictors of Nonresponse to Directly Acting Antiviral Therapy in Patients With Chronic Hepatitis C and Decompensated Cirrhosis", OPEN FORUM INFECTIOUS DISEASES, vol. 4, no. 2, 1 April 2017 (2017-04-01), XP055388688, DOI: 10.1093/ofid/ofx067 * p 2, col 1, para 5)(p 2, col 2, para 4)(p 3, col 1, para 1) * | 1-10 | |

-/--

The present search report has been drawn up for all claims

| Place of search | Date of completion of the search | Examiner |
|---|---|---|
| Munich | 7 July 2017 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt

European Patent Office

Office européen des brevets

# EUROPEAN SEARCH REPORT

Application Number

EP 17 16 6600

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Syed I Rahman ET AL: "Predictors of Clinical Response in Hepatitis C DAA Therapy", , 1 April 2016 (2016-04-01), pages S-1103, XP055388700, Retrieved from the Internet: URL:http://ac.els-cdn.com/S001650851633727 1/1-s2.0-S0016508516337271-main.pdf?_tid=b 7a8a584-6257-11e7-ae70-00000aacb360&acdnat =1499351634_a1bdf4a370cddf306fd404273d7304 d2 [retrieved on 2017-07-06] * abstract * ----- | 1-10 | |
| X | AARON F. CARLIN ET AL: "Temporal dynamics of inflammatory cytokines/chemokines during sofosbuvir and ribavirin therapy for genotype 2 and 3 hepatitis C infection : HEPATOLOGY, Vol. XX, No. X, 2015", HEPATOLOGY, vol. 62, no. 4, 22 October 2015 (2015-10-22), pages 1047-1058, XP055387519, ISSN: 0270-9139, DOI: 10.1002/hep.27971 * (p 1051, col 1, end of first para) * ----- | 1-10 | TECHNICAL FIELDS SEARCHED (IPC) |
| Y | AGHEMO ALESSIO ET AL: "Response-Guided Duration of Direct Acting Antiviral Therapy for Chronic Hepatitis C: Back to the Future?", GASTROENTEROLOGY, ELSEVIER, AMSTERDAM, NL, vol. 152, no. 5, 28 February 2017 (2017-02-28), pages 1238-1239, XP029955964, ISSN: 0016-5085, DOI: 10.1053/J.GASTRO.2017.02.022 * (p 1238, col 1-2, bridging para). (p 1238, col 1, para 1) * ----- | 11-16 | |

The present search report has been drawn up for all claims

| Place of search | Date of completion of the search | Examiner |
|---|---|---|
| Munich | 7 July 2017 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WHITLOCK ; SCHLUTER.** The analysis of biological data. June 2014

- **COLOMBO S. et al.** *J Gastroenterol,* 2012, vol. 47, 461-469